# EUROPÄISCHE PATENTSCHRIFT

(11) **EP 2 012 699 B1**
(45) Veröffentlichungstag und Bekanntmachung des Hinweises auf die Patenterteilung: **31.03.2010**
(21) Anmeldenummer: 07724589.2
(22) Anmeldetag: 25.04.2007
(51) Int. Cl.: A61B 19/00

(54) **VORRICHTUNG ZUR BEHANDLUNG VON MENSCHLICHEM ODER TIERISCHEM GEWEBE MITTELS EINES MAGNETISCH BEWEGBAREN INSTRUMENTS**
DEVICE FOR TREATMENT OF HUMAN OR ANIMAL TISSUE BY MEANS OF A MAGNETICALLY MOVABLE INSTRUMENT
PROCÉDÉ DE TRAITEMENT DE TISSU HUMAIN OU ANIMAL AU MOYEN D'UN INSTRUMENT À DÉPLACEMENT MAGNÉTIQUE

(30) Priorität: 27.04.2006 DE 102006019680; 27.06.2006 DE 102006029455
(43) Veröffentlichungstag der Anmeldung: 14.01.2009
(73) Patentinhaber: Erbe Elektromedizin GmbH, 72072 Tübingen (DE)
(72) Erfinder: HAGG, Martin, 72827 Wannweil (DE)
(74) Vertreter: Bohnenberger, Johannes
(86) Internationale Anmeldenummer: PCT/EP2007/003659
(87) Internationale Veröffentlichungsnummer: WO 2007/124909

(56) Entgegenhaltungen:
- WO-A-99/18852
- US-A- 3 358 676
- US-A1- 2002 103 430
- US-A1- 2004 019 447
- US-A1- 2005 182 315

## Beschreibung

Die Erfindung betrifft eine Vorrichtung zur Behandlung von menschlichem oder tierischem Gewebe.

Bei der Behandlung von menschlichem oder tierischem Gewebe, insbesondere bei minimalinväsiven Operationen oder auch bei Therapien, werden oftmals Instrumente in den Körper eingeführt und müssen an einen Applikationsort bewegt werden. Zusätzlich gilt es oftmals, am Applikationsort eine mechanische Arbeit zu verrichten. Typische Beispiele hierfür sind die Devitalisierung und/oder Entfernung von Tumorgewebe. Ein anderes typisches Beispiel ist die Behandlung einer Cholezystolithiasis (Gallenblasenstein) oder die Entfernung von Nephrolithen aus Nierengängen oder ableitenden Harnwegen. In manchen Fällen ist hierbei das Arbeiten mit einem Endoskop problematisch, da das Bewegen des eigentlichen, an dem distalen Ende des Endoskops sitzenden Instruments Schwierigkeiten bereitet. WO 99/18852 und US 2002/0103430 offenbaren Instrumente, die mit Hilfe eines magnetresonanztomographen zum Zielort navigiert werden. Beide Schriften offenbaren den Oberbegriff des Ansprüche 1 und 2.

Der Erfindung liegt die Aufgabe zu Grunde, eine Vorrichtung der eingangs genannten Art dahin gehend aufzuzeigen, dass die Bewegung eines Instruments innerhalb eines Körpers oder eines Gewebebereiches ohne mechanische Verbindung nach außen ermöglicht wird.

Diese Aufgabe wird durch eine Vorrichtung nach Anspruch 1 bzw. 2 gelöst.

Ein wesentlicher Punkt der Erfindung liegt darin, dass mittels eines auf dem Markt verfügbaren Gerätes, insbesondere eines Magnetresonanztomographen (Kernspintomographen) ein starkes, statisches Magnetfeld erzeugt wird, das man dazu ausnützt, in Zusammenwirkung mit einem gesondert erzeugten Magnetfeld einer stromdurchflossenen Leiterschleife eine Kraft zu erzeugen, welche das Instrument beaufschlagt. Diese Kraft wiederum kann zur Bewegung des Instrumentes innerhalb des Körpers bzw. Gewebes, aber auch zur Verrichtung von mechanischer Arbeit am Gewebe benutzt werden.

Ein weiterer wichtiger Gesichtspunkt liegt hierbei darin, dass, sofern ein MRT (Magnetresonanztomograph) verwendet wird, die gesamte Behandlung gleichzeitig durch bildgebende Verfahren beobachtet werden kann. Es werden also hier "zwei Fliegen mit einer Klappe geschlagen", da das Magnetfeld einerseits Grundlage des bildgebenden Verfahrens bildet, andererseits zur Erzeugung mechanischer Arbeit ausgenützt wird.

Insbesondere wird die oben genannte Aufgabe durch eine Vorrichtung zur Behandlung von menschlichem oder tierischem Gewebe mittels eines bewegbaren Instruments gelöst, umfassend eine Einrichtung zur Erzeugung eines magnetischen Stator-Feldes, in welchem sich das Gewebe befindet, insbesondere einen Magnetresonanztomographen (MRT), mindestens eine Leiterschleife an oder im Instrument, sowie einen Generator zum Erzeugen eines, die Leiterschleife durchfließenden Stroms zur Erzeugung eines magnetischen Nutz-Feldes, das in Zusammenwirkung mit dem Stator-Feld eine resultierende Wirkung zur Beaufschlagung des Instrumentes mit einer Nutzkraft erzeugt.

Grundsätzlich kann auch eine explizite Einrichtung zur Erzeugung des erforderlichen Stator-Feldes eingesetzt werden, um die erfindungsgemäße Vorrichtung zu realisieren. Die Einrichtung zur Erzeugung des Magnetfeldes ist also nicht auf einen MRT beschränkt.

Vorzugsweise wird eine Vielzahl von zueinander jeweils in einem bestimmten Raumwinkel, insbesondere drei zueinander in einem Raumwinkel von 90° angeordneten Leiterschleifen vorgesehen. Weiterhin ist der Generator zur Bestromung der Leiterschleifen derart vorgesehen und ausgebildet, dass die Nutzkraft hinsichtlich ihrer Richtung und Größe einstellbar ist. Dadurch ist ein verbessertes "Manövrieren" des Instrumentes möglich.

Bei einer bevorzugten Ausführungsform ist der Generator zur Erzeugung eines Wechselstromes ausgebildet, so dass das Instrument Schwingungen ausführen kann. Hierbei kann es von Vorteil sein, den Generator derart auszubilden, dass der Wechselstrom hinsichtlich seiner Frequenz auf eine Resonanzfrequenz des Instrumentes einstellbar ist. Durch Ausnützung einer solchen mechanischen Resonanz ist es möglich, auch bei geringer Energiezufuhr relativ hohe Schwingungsamplituden zu erzeugen. Wenn hierbei der Generator zur Lieferung einer zusätzlichen Gleichstromkomponente ausgebildet ist, kann ein "Netto-Vortrieb" erzeugt werden, so dass beispielsweise das Instrument einerseits keine Haftreibung überwinden muss, andererseits aber eine Vortriebskomponente erfährt, so dass es zu einem Zielort manövrierbar ist.

Alternativ (oder auch zusätzlich) ist es möglich, den Generator zur Erzeugung eines Drehfeldes und das Instrument derart auszubilden, dass dessen befindliche bohrende oder fräsende Teile in Drehung versetzbar sind. Auch hier können natürlich wiederum verschiedene Bewegungs- bzw. Kraftkomponenten derart zusammentreffen, dass zusätzlich zu einer drehenden Bewegung eine schwingende (schlagende) Bewegung kommt.

Als erfinderisch wird es auch betrachtet, einen Magnetresonanztomographen zur Erzeugung eines Stator-Feldes für eine elektrische Maschine zum Antrieb eines chirurgischen oder therapeutischen Instruments zu verwenden. Die Vorteile hiervon wurden oben bereits dargestellt.

Nachfolgend werden Ausführungsbeispiele der Erfindung anhand von Abbildungen näher erläutert. Hierbei zeigen:
- - Fig. 1: eine erste bevorzugte Ausführungsform der Erfindung in schematisierter Darstellung in ihrer "Arbeitsumgebung" und
- - Fig. 2: eine zweite Ausführungsform der Erfindung in einer schematisierten Darstellung.

In der nachfolgenden Beschreibung werden für gleiche und gleich wirkende Teile dieselben Bezugsziffern verwendet.

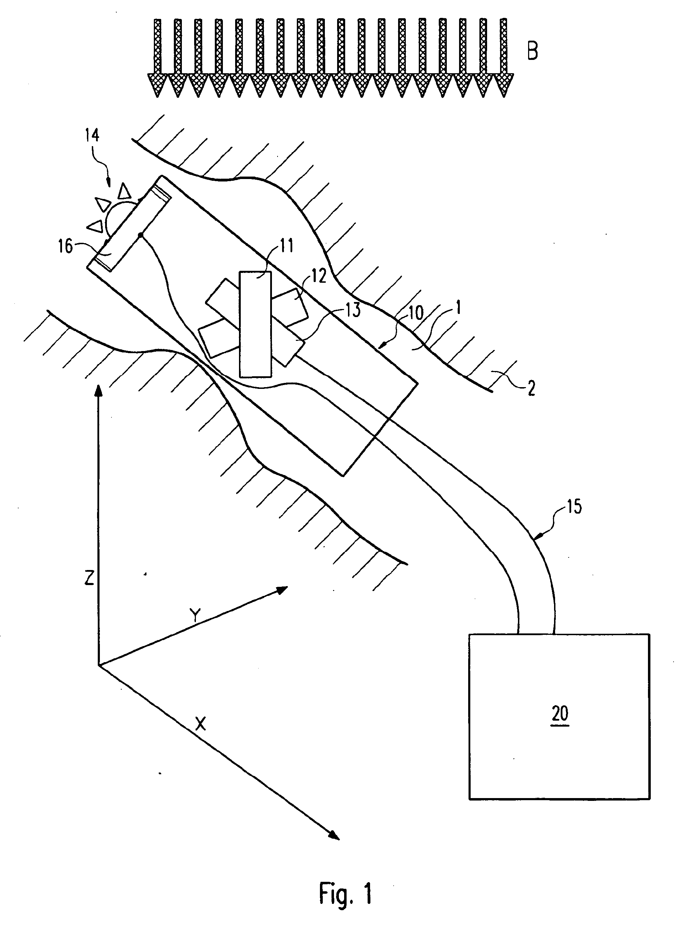

In der Fig. 1 ist mit B ein Magnetfeld schematisiert dargestellt, das von einem Magnetresonanztomographen erzeugt wird. Im Magnetfeld B befindet sich ein Patient, von dessen Gewebe 2 ein Bereich mit einem Hohlorgan 1, z.B. einem Harnleiter dargestellt ist.

Es sei darauf hingewiesen, dass das Magnetfeld auch von einer explizit hierfür vorgesehenen Einrichtung erzeugt werden kann.

Im Hohlorgan 1 befindet sich ein Instrument 10, in dessen Innerem drei als Spulen ausgebildete Leiterschleifen 11,12 und 13 angeordnet sind. Die Richtungen dieser Spulen 11 - 13 sind in der Abbildung im Koordinatensystem X, Y, Z schematisiert wiedergegeben.

Die drei Leiterschleifen bzw. Spulen 11 - 13 sind über eine Zuleitung 15 mit einem Generator 20 verbunden, der jede der Leiterschleifen 11 - 13 individuell bestromen kann, so dass sich in Zusammenwirkung mit dem statischen Magnetfeld B eine resultierende Kraft auf das Instrument 10 ausbildet. Im vorliegenden Fall wäre es beispielsweise vorstellbar, die Leiterschleifen 11 - 13 derart durch den Generator 10 zu bestromen, dass das Instrument 10 einerseits eine Kraftkomponente in X-Richtung (also in Richtung des Hohlorgans 1) erfährt, andererseits dieser gleichförmigen Kraft eine Schwingung überlagert ist, welche das Instrument 10 samt einem an seiner Spitze befindlichen Fräskopf 14 in eine Schwingung in Bewegungsrichtung versetzt. Zusätzlich hierzu kann ein Drehmoment durch Aufbringung eines entsprechenden Drehfeldes auf die Leiterschleifen 11 - 13 erzeugt werden, so dass sich das Instrument 10 (ggf. auch nur der Fräskopf 14) dreht. Auf diese Weise könnte beispielsweise ein im Hohlorgan 1 befindlicher Gegenstand (z.B. ein Nierenstein) zertrümmert werden.

Zusätzlich zur Erzeugung der Kraftwirkung unter Zuhilfenahme des statischen Feldes B kann im Instrument 10 ein (weiterer) elektromechanischer Wandler 16 vorgesehen sein, z.B. ein Ultraschallschwinger, der ebenfalls über den Generator 20 bestromt wird und so den Fräskopf 14 in (Ultraschall-) Schwingungen versetzt.

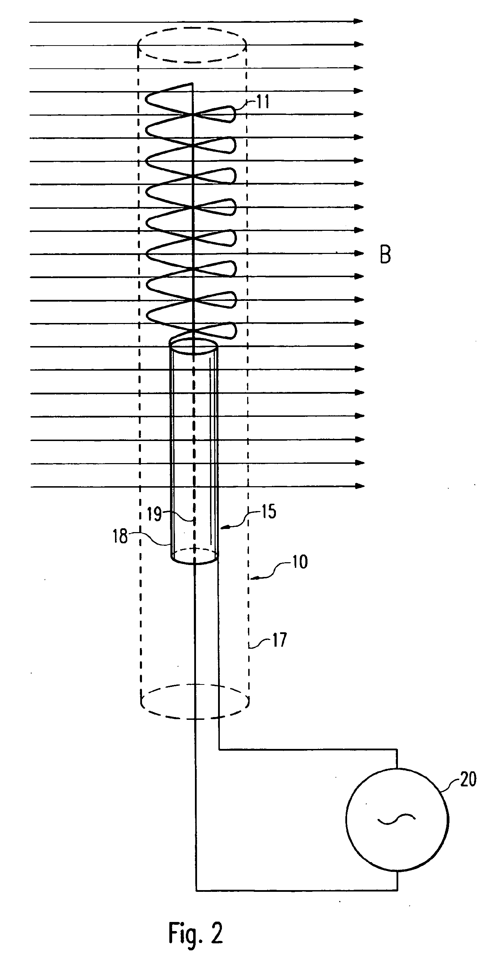

Bei dem in Fig. 2 gezeigten Ausführungsbeispiel der Erfindung besteht das Instrument aus einem stabförmigen Schaft 17 aus nichtmagnetischem Material, an dem in einem aktiven Bereich (in Fig. 2 am oberen Ende) die erste Leiterschleife 11 spulenförmig angebracht ist. Diese Spule wird durch eine koaxiale Zuleitung 15 mit einem Außenleiter 18 und einem Innenleiter 19 vom Generator 20 mit Wechselstrom versorgt, während sich der "aktive Bereich" innerhalb des im Kernspin-Tomographen vorhandenen permanenten und zumindest in diesem Bereich als homogen zu sehenden Magnetfeld B befindet.

Entsprechend der momentanen Stromrichtung wird der "aktive Bereich" somit ein Drehmoment in oder entgegen dem Uhrzeigersinn erfahren und aufgrund der Elastizität des Schaftes 17 (und aller darin angebrachten Vorrichtungsteile) in die entsprechende Richtung ausgelenkt werden. Die Größe der Auslenkung ist abhängig von der Elastizität des Schaftes 17, der Stärke des Stroms, der Stärke des Magnetfelds und des Winkels, den die Achse der Leiterschleife 11 zur Richtung der Magnetfeldlinien einnimmt.

Durch einen durch diese Anordnung fließenden Wechselstrom kann der "aktive Bereich" folglich zum Schwingen gebracht werden. Besonders hohe Amplituden bei relativ geringer Stromstärke können bei geeigneter Wahl der Stromfrequenz derart erzeugt werden, dass diese der Resonanzfrequenz der Anordnung entsprechen, welche durch die Elastizität des Schaftes und der Massenverteilung innerhalb des Schaftes (aktiver Bereich zu inaktiver Bereich) definiert ist.

Die Zuleitung 15 wird vorzugsweise außerhalb des "aktiven Bereichs" koaxial aufgebaut, damit die durch den Stromfluss im Magnetfeld entstehenden Lorentzkräfte sich gegenseitig aufheben.

Bei einer hier nicht in der Abbildung gezeigten Ausführungsform wird das Instrument als nadelförmige Sonde gebildet, so dass der "aktive Bereich" in das zu behandelnde Zielgewebe eingeführt werden kann. Somit können die erzeugten Schwingungen in das an- und umliegende Gewebe übertragen werden, so dass dort die mechanisch erzeugte Energie in Wärmeenergie umgewandelt wird. Diese Wärme kann genutzt werden, um beispielsweise Tumorgewebe gezielt thermisch zu devitalisieren. Weiterhin besteht eine Möglichkeit auch darin, durch die Schwingungen entsprechender Frequenz die Zellstrukturen des Zielgewebes mechanisch zu zerstören, um damit eine Devitalisierung oder Defragmentierung des Gewebes zu erzeugen.

Aus Obigem ergibt sich, dass die verschiedenen Einzelgedanken, die im Zusammenhang mit den beiden Figurenbeschreibungen ausgeführt wurden, auch kombiniert werden können.

### Bezugszeichenliste

- 1: Hohlorgan
- 2: Gewebe
- 10: Instrument
- 11: erste Leiterschleife
- 12: zweite Leiterschleife
- 13: dritte Leiterschleife
- 14: Fräskopf
- 15: Zuleitung
- 16: Wandler
- 20: Generator

## Patentansprüche

1. Vorrichtung zur chirurgischen Behandlung von menschlichem oder tierischem Gewebe mittels eines bewegbaren Instruments (10), umfassend
eine Einrichtung zur Erzeugung eines magnetischen Stator-Feldes (B), in welchem sich das Gewebe (2) befindet, insbesondere einen Magnetresonanztomographen (MRT);
mindestens eine Leiterschleife (11 - 13) an oder in dem Instrument (10),
sowie einen Generator (20) zur Erzeugung eines die Leiterschleife (11 - 13) durchfließenden Stroms zum Erzeugen eines magnetischen Nutz-Feldes, das in Zusammenwirkung mit dem Stator-Feld (B) eine resultierende Wirkung zur Beaufschlagung des Instruments (10) mit einer Nutzkraft derart erzeugt, dass das Instrument mechanische Arbeit am Gewebe verrichtet,
**dadurch gekennzeichnet, dass**
das Instrument bohrende oder fräsende Teile aufweist und der Generator (20) zur Erzeugung eines Drehfeldes und das Instrument (10) derart ausgebildet sind, dass dessen bohrende oder fräsende Teile (14) in Drehung versetzbar sind.

2. Vorrichtung zur chirurgischen Behandlung von menschlichem oder tierischem Gewebe mittels eines bewegbaren Instruments (10), umfassend
eine Einrichtung zur Erzeugung eines magnetischen Stator-Feldes (B), in welchem sich das Gewebe (2) befindet, insbesondere einen Magnetresonanztomographen (MRT);
mindestens eine Leiterschleife (11 - 13) an oder in dem Instrument (10),
sowie einen Generator (20) zur Erzeugung eines die Leiterschleife (11 - 13) durchfließenden Stroms zum Erzeugen eines magnetischen Nutz-Feldes, das in Zusammenwirkung mit dem Stator-Feld (B) eine resultierende Wirkung zur Beaufschlagung des Instruments (10) mit einer Nutzkraft erzeugt, **dadurch gekennzeichnet, dass** die Nutzkraft derart erzeugt wird, dass das Instrument Schwingungen auf das Gewebe überträgt, so dass mechanische Energie in Wärmeenergie umgewandelt wird, wodurch Tumorgewebe gezielt thermisch devitalisiert wird.

3. Vorrichtung nach Anspruch 1 oder 2,
**dadurch gekennzeichnet, dass**
eine Vielzahl von zueinander jeweils in einem bestimmten Raumwinkel, insbesondere drei zueinander in einem Raumwinkel von 90° angeordneten Leiterschleifen (11 - 13), sowie der Generator (20) zur Bestromung der Leiterschleifen (11 - 13) derart vorgesehen und ausgebildet sind, dass die Nutzkraft hinsichtlich ihrer Richtung und Größe einstellbar ist.

4. Vorrichtung nach einem der vorhergehenden Ansprüche,
**dadurch gekennzeichnet, dass**
der Generator (20) zur Erzeugung eines Wechselstromes ausgebildet ist.

5. Vorrichtung nach Anspruch 4,
**dadurch, gekennzeichnet, dass**
der Generator (20) derart ausgebildet ist, dass der Wechselstrom hinsichtlich seiner Frequenz auf eine Resonanzfrequenz des Instruments (10) einstellbar ist.

6. Vorrichtung nach einem der Ansprüche 4 oder 5,
**dadurch gekennzeichnet, dass**
der Generator (20) zur Lieferung einer zusätzlichen Gleichstromkomponente ausgebildet ist.

## Claims

1. Device for surgical treatment of human or animal tissue by means of a movable instrument (10) comprising
a means for generating a magnetic stator field (B), in which the tissue (2) is located, in particular a magnetic resonance tomograph (MRT);
at least one conductor loop (11 - 13) on or in the instrument (10),
and a generator (20) for generating a current that flows though the conductor loop (11 - 13) in order to generate a magnetic useful field, which, in conjunction with the stator field (B) generates a resulting action for applying useful force to the instrument (10) in such a way that the instrument performs mechanical work on the tissue
**characterised in that**
the instrument comprises drilling or milling parts and the generator (20) for generating a rotating field and the instrument (10) are designed in such a way that its drilling or milling parts (14) can be caused to rotate thereby.

2. Device for surgical treatment of human or animal tissue by means of a movable instrument (10) comprising
a means for generating a magnetic stator field (B), in which the tissue (2) is located, in particular a magnetic resonance tomograph (MRT);
at least one conductor loop (11 - 13) on or in the instrument (10),
and a generator (20) for generating a current that flows though the conductor loop (11 - 13) in order to generate a magnetic useful field, which, in conjunction with the stator field (B) generates a resulting action for applying useful force to the instrument (10)
**characterised in that** the useful force is generated in such a way that the instrument transfers vibrations to the tissue so that mechanical energy is converted into thermal energy which causes tumorous tissue to be selectively thermally devitalised.

3. Device according to claim 1 or claim 2,
**characterised in that**
a plurality of conductor loops (11 - 13) arranged respectively at a certain solid angle with respect to each other, in particular three conductor loops arranged at a solid angle of 90° with respect to each other, and the generator (20) for supplying a current that passes through the conductor loops (11 - 13) are provided and designed in such a way that the direction and magnitude of the useful force can be adjusted.

4. Device according to any one of the preceding claims,
**characterised in that**
the generator (20) is designed to generate an alternating current.

5. Device according to claim 4,
**characterised in that**
the generator (20) is designed in such a way that the frequency of the alternating current may be adjusted to a resonance frequency of the instrument (10).

6. Device according to one of claims 4 or 5,
**characterised in that**
the generator (20) is designed to supply an additional direct current component.

## Revendications

1. Dispositif de traitement chirurgical de tissu humain ou animal au moyen d'un instrument mobile (10), comprenant :
une installation de production d'un champ magnétique statorique (B), dans lequel se trouve le tissu (2), en particulier un tomographe à résonance magnétique (MRT) ;
au moins une boucle conductrice (11 à 13) au niveau de ou dans l'instrument (10),
ainsi qu'un générateur (20) pour la production d'un courant traversant la boucle conductrice (11 à 13) en vue de produire un champ magnétique qui produit une action résultante en collaboration avec le champ statorique (B) en vue de l'alimentation de l'instrument (10) avec une force utile, de sorte que l'instrument exécute un travail mécanique au niveau du tissu,
**caractérisé en ce que**
l'instrument présente des pièces de forage ou de fraisage et le générateur (20) destiné à la production d'un champ rotatif et l'instrument (10) sont réalisés de manière à ce que les pièces (14) de forage ou de fraisage de ce dernier puissent être déplacées en rotation.

2. Dispositif de traitement chirurgical de tissu humain ou animal au moyen d'un instrument mobile (10), comprenant :
une installation de production d'un champ magnétique statorique (B), dans lequel se trouve le tissu (2), en particulier un tomographe à résonance magnétique (MRT) ;
au moins une boucle conductrice (11 à 13) au niveau de ou dans l'instrument (10),
ainsi qu'un générateur (20) pour la production d'un courant traversant la boucle conductrice (11 à 13) en vue de produire un champ magnétique qui produit une action résultante en collaboration avec le champ statorique (B) en vue de l'alimentation de l'instrument (10) avec une force utile,
**caractérisé en ce que**
la force utile est produite de sorte que l'instrument transmet des vibrations au tissu, de sorte que de l'énergie mécanique est convertie en énergie thermique, ce qui provoque une dévitalisation thermiquement ciblée de tissu tumoral.

3. Dispositif selon la revendication 1 ou 2,
**caractérisé en ce que**
une pluralité de boucles conductrices (11 à 13) disposées ensemble respectivement selon un angle solide déterminé, en particulier trois boucles conductrices (11 à 13) disposées selon un angle solide de 90°, ainsi que le générateur (20) destiné à l'alimentation de courant des boucles conductrices (11 à 13) sont prévues et réalisées de sorte que la force utile puisse être ajustée en ce qui concerne sa direction et sa taille.

4. Dispositif selon l'une quelconque des revendications précédentes,
**caractérisé en ce que**
le générateur (20) est réalisé en vue de la production d'un courant alternatif.

5. Dispositif selon la revendication 4,
**caractérisé en ce que**
le générateur (20) est réalisé de manière à ce que la fréquence du courant alternatif puisse être ajustée à une fréquence de résonance de l'instrument (10).

6. Dispositif selon la revendication 4 ou 5,
**caractérisé en ce que**
le générateur (20) est réalisé en vue de la fourniture d'une composante supplémentaire de courant continu.
